# EUROPÄISCHE PATENTANMELDUNG

(11) **EP 1 035 414 A2**
(43) Veröffentlichungstag der Anmeldung: **13.09.2000**
(21) Anmeldenummer: 00103912.2
(22) Anmeldetag: 24.02.2000
(51) Int. Cl.: G01N 33/566, G01N 33/577, G01N 33/58, G01N 33/543, G01N 33/66

(54) **Inverser Lectin-Assay (ILA) zur Identifizierung und/oder Quantifizierung einer Kohlenhydrat-Bindeaktivität**

(30) Priorität: 05.03.1999 DE 99103922
(71) Anmelder: MADAUS AG, D-51109 Köln (DE)
(72) Erfinder: Lentzen, Hans Prof. Dr, 51503 Rösrath (DE); Witthohn, Klaus Dr, 51491 Overath (DE)
(74) Vertreter: VOSSIUS & PARTNER

(57) **Zusammenfassung**

Die Erfindung betrifft ein Verfahren zur Identifizierung und/oder Quantifizierung einer ersten Substanz, welche Kohlenhydrat bindet, das die Schritte (a) Zugabe einer zu testenden Probe zu einem Trägermaterial, wobei das Trägermaterial einen immobilisierten Rezeptor trägt, welcher einen Teil der ersten Substanz spezifisch bindet, der nicht in die Kohlenhydratbindung involviert ist; (b) Zugabe einer zweiten Substanz, die einen Kohlenhydrat-Anteil enthält, wobei der Kohlenhydrat-Anteil durch die erste Substanz in einem Komplex gebunden wird und wobei der Komplex nachweisbar ist; und (c) Nachweis des Komplexes umfaßt. Vorzugsweise ist die zweite Substanz, die den Kohlenhydratanteil enthält, ein Mistellektin. Ferner betrifft die Erfindung einen Test-Kit zur Ausführung des Verfahrens, welcher (a) einen Rezeptor, welcher einen Teil der ersten Substanz spezifisch bindet, der nicht in die Kohlenhydratbindung involviert ist; (b) eine zweite Substanz, wie vorstehend definiert; und (c) ein Detektionssystem zum Nachweis der zweiten Substanz enthält.

## Beschreibung

Die Erfindung betrifft ein Verfahren zur Identifizierung und/oder Quantifizierung einer ersten Substanz, weiche Kohlenhydrat bindet, das die Schritte (a) Zugabe einer zu testenden Probe zu einem Trägermaterial, wobei das Trägermaterial einen immobilisierten Rezeptor trägt, welcher einen Teil der ersten Substanz spezifisch bindet, der nicht in die Kohlenhydratbindung involviert ist; (b) Zugabe einer zweiten Substanz, die einen Kohlenhydrat-Anteil enthält, wobei der Kohlenhydrat-Anteil durch die erste Substanz in einem Komplex gebunden wird und wobei der Komplex nachweisbar ist; und (c) Nachweis des Komplexes umfaßt. Vorzugsweise ist die zweite Substanz, die den Kohlenhydratanteil enthält, ein Mistellektin. Ferner betrifft die Erfindung einen Test-Kit zur Ausführung des Verfahrens, welcher (a) einen Rezeptor, welcher einen Teil der ersten Substanz spezifisch bindet, der nicht in die Kohlenhydratbindung involviert ist; (b) eine zweite Substanz, wie vorstehend definiert; und (c) ein Detektionssystem zum Nachweis der zweiten Substanz enthält.

Eine Aktivitätscharakterisierung von Molekülen mit Kohlenhydrat-Bindeaktivität (Leguminosen-, Cerealien-, C-Typ- oder S-Typ-Lektinen (Sharon, 1993); Typ II-Ribosomen-inaktivierenden Proteinen; Antikörpern mit Kohlenhydrat-Bindeaktivität) ist insbesondere zur Quantifizierung und Identifizierung von therapeutisch interessanten Lektin-Molekülen von breitem Interesse. Für einige solcher Moleküle mit Kohlenhydrat-Bindeaktivität werden anticancerogene oder cytotoxische Aktivitäten beschrieben.

Im Stand der Technik ist eine Reihe von Lektinen bekannt, so zum Beispiel Ricin oder das Mistellektin. Mistelextrakte werden schon seit vielen Jahrzehnten in der adjuvanten Tumortherapie eingesetzt. Mistellektin (ML), ein potentes Toxin aus der Mistel, konnte Ende der 80er Jahre als die aktive Komponente der Mistelextrakte beschrieben werden (Haito et al. 1989). Mistellektin ist ein Heterodimer (es gehört der RIP II-Proteinfamilie an), von dem die beiden Einzelketten zwei vollkommen unterschiedliche Aktivitäten aufweisen. Die A-Kette weist eine enzymatische Aktivität (N-Glycosidase-Aktivität) auf, die B-Kette hingegen eine Kohlenhydrat-Bindeaktivität (Lektin). Eine Internalisierung in eine Zielzelle erfolgt über die Kohlenhydrat-bindende B-Kette über einen noch nicht identifizierten glycosylierten Rezeptor. In einem MOLT-4 Zytotoxizitätstest (Möckel et al., 1997) kommt es bei gleichzeitiger Inkubation des Mistellektins mit Galactose oder Lactose an der Zielzelle zu einer Erhöhung des IC₅₀ Wertes um den Faktor 10, was zeigt, daß für den ersten Kontakt zwischen Mistellektin und Zielzelle die Kohlenhydrat-Bindung verantwortlich ist. Nach erfolgreicher Internalisierung von an den Rezeptor gebundenem Mistellektin und anschließender proteolytischer Spaltung der enzymatisch aktiven A-Kette von der B-Kette sowie dem Transfer der A-Kette durch die endosomale Membran ins Cytoplasma werden an den Ribosomen die 28S rRNA Moleküle durch Abspaltung eines bestimmten Adenins inaktiviert (Stirpe et al., 1992). Die beschriebenen Aktivitäten des Mistellektins wie z.B. Aktivierung von verschiedenen Zellpopulationen wie den "Large Granular Lymphocytes" (LGL) oder von natürlichen Killerzellen, (NK Zellen) (Hajto et al., 1989) sowie Induktion von Cytokinen wie TNFα und IFN γ (Möckel et al., 1997) oder Oberflächenmarkern wie CD-25 und HLA-DQ (Beuth et al., 1992) sind dosisabhängig. Die Abhängigkeit von der Dosis kann durch eine Gaußsche Verteilung beschrieben werden. Für eine genaue Dosierung des Wirkstoffes ist es daher nötig, die Aktivität des Wirkstoffes, die sich über die Zellbindung vermittelnde Kohlenhydrat-Bindung definiert, zu quantifizieren.

Von einigen Herstellern werden schon heute Mistelextraktpräparate auf einen definierten Mistellektin (ML)-Gehalt eingestellt (Lektinol, MADAUS AG; Eurixor, Medisculab Arzneimittel GmbH). Hier wird der Gehalt an Mistellektin über den Bezug zu einer Referenzsubstanz in ng/ml angegeben. Da die spezifische Aktivität des Wirkstoffes variieren kann, ist diese Gehaltsangabe als suboptimal anzusehen. Anstelle eines Gehaltes an Wirkstoff (in ng/ml) bezogen auf eine Referenzsubstanz ist es von Interesse, eine Dosierung des Wirkstoffes über seine Kohlenhydrat-Bindeaktivität zu beschreiben.

Zur Bestimmung des Gehaltes an Molekülen mit Lektin-Aktivität insbesondere des ML-Gehaltes in Mistelextraktpräparaten wird bisher standardmäßig der Enzyme-Linked-Lectin-Assay (ELLA) eingesetzt (Vang et al., 1986). Hierbei werden auf eine mit einer Glykoprotein-Matrix (z.B. Asialofetuin oder Fetuin) beschichteten Mikrotiterplatte mit 96 Vertiefungen eine ML-Standardverdünnungsreihe und die zu analysierenden unbekannten Proben pipettiert und dann inkubiert. Im weiteren Verlauf des Tests wird über die Kohlenhydrat-Wechselwirkung gebundenes ML über eine Antigen Antikörper-Reaktion mit spezifischen Antikörpern und diese Wechselwirkung mit einem an Peroxidase-gekoppelten Zweitantikörper, der gegen den Fc-Teil des Anti-ML-Antiserums gerichtet ist, nachgewiesen.
Eine Quantifizierung des Mistellektins erfolgt colorimetrisch durch Substratumsetzung über den Bezug der Absorption der unbekannten Proben mit der Absorption der mitgeführten ML-Standardverdünnungsreihe.

Von Schöllhorn (Patent DE 4123263 A1) wurde der ELLA Test dahingehend modifiziert, daß anstelle des Glykoproteins Asialofetuin zum Beschichten der Platten ein Neoglycoprotein (an Rinderserum-Albumin gekoppelte Kohlenhydrate wie z.B. N-Acetyl-Neuraminsäure, N-Acetyl-Galactosid oder Galactose) eingesetzt wird. Dadurch gelingt dem Autor eine Diskriminierung verschiedener Mistellektin-Isoformen (insbesondere ML-I und ML-II) abhängig von dem eingesetzten Neoglycoprotein. Eine Gehaltsbestimmung wurde auch von Schöllhorn über einen mitgeführten Standard erreicht.

Hatakeyama et al. (1996) beschreiben einen ELLA-ähnlichen Test, in dem anstelle des Asialofetuins definierte Kohlenhydrate chemisch über Divinylsulfon an die Mikrotiterplatte gebunden wurden. Die Bindung mehrerer verschiedener Lektine (*Ricinus communis* Agglutinin, Concanavalin A, Weizenkeim-Agglutinin) an diese definierten Matrices und die anschließende Detektion gebundenen Lektins mit colloidaler Gold-Lösung im Protein-Test (Absorption bei 620 nm) führte zwar zu einer mengenabhängigen Zunahme der optischen Dichte bei 620 nm, doch gelang es den Autoren bis hin zu einer Lektin-Konzentration von 300 µg/ml nicht, das System in eine Sättigung zu überführen, was mit der hohen beobachteten Dissoziation des Lektins von der Kohlenhydrat-Matrix erklärt wurde.

Duk et al. (1994) beschreiben einen ELLA, in dem sie Glycophorin A aus humanen Erythrocyten oder in der Glycosylierung chemisch modifizierte Glycophorine zum Beschichten der Festphase einsetzen. Dieses System wurde herangezogen, um die unterschiedliche Kohlenhydrat-Affinität verschiedener biotinylierter Lektine, die an Avidin-Alkalische Phosphatase gekoppelt waren, zu beschreiben. Den Autoren gelingt es bei einem Einsatz von bis zu 8 µg Lektin nicht, eine systeminterne Sättigung zu erlangen.
Dieser Test kann daher nur zur Beschreibung von Spezifitäten, jedoch nicht zur Volumenaktivitätsbestimmung eingesetzt werden.

Die oben beschriebenen Testsysteme haben diverse Nachteile. Ein Vergleich von verschiedenen Chargen kann nur über eine gut charakterisierte Standardcharge erfolgen. Das Ergebnis (in ng/ml) wird in 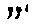 Äquivalenten der Standardcharge" ausgedrückt. Es besteht daher die Notwendigkeit, über die gesamte Dauer einer Produktlaufzeit eine Standardcharge zu definieren und vorzuhalten. Dabei muß gewährleistet sein, daß die Aktivität der Standardcharge über die Zeit stabil bleibt, um eine veränderte Dosierung in 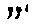 Standardchargen-Äquivalenten" zu vermeiden. Herstellerbedingt gibt es jedoch allein auf dem deutschen Markt mehrere verschiedene Standardchargen, die einen Vergleich unterschiedlicher Präparate nur schwer erlauben.

Als nachteilig wirkt sich bei den vorgenannten, aus dem Stand der Technik bekannten Verfahren ferner aus, daß bei einer Analyse von Extraktpräparaten der tatsächliche Gehalt an Molekülen mit Lektin-Aktivität, insbesondere der Mistellektin-Gehalt, aufgrund potentieller Verunreinigungen durch z.B. kompetitierende Kohlenhydrate (Galaktose / Lactose / Oligosaccharide), die die Wechselwirkung zwischen Matrix und Ligand beeinflussen, nicht erfaßt werden kann, bzw. "falsch negative" Ergebnisse liefert. Hinzu kommt, daß im ELLA polyklonale Anti-ML-Antiseren zur Detektion gebundener Mistellektine eingesetzt werden, wodurch zusätzlich die Gefahr von Kreuzreaktionen und somit die Gefahr von "falsch positiven" Ergebnissen besteht. Unerwünscht ist insbesondere die wenig affine Wechselwirkung zwischen Lektinen und den eingesetzten immobilisierten Rezeptoren, wie Asialofetuin oder Neoglycoproteinen.

Aufgabe der vorliegenden Erfindung war somit, die vorgenannten nachteiligen Verfahren aus dem Stand der Technik zu verbessern und ein zuverlässiges Verfahren zur Isolierung/Identifizierung und/oder Quantifizierung von Substanzen mit Kohlenhydrat-Bindeaktivität bereitzustellen.

Diese Aufgabe wird durch die in den Ansprüchen gekennzeichneten Ausführungsformen gelöst.

Somit betrifft die Erfindung Verfahren zur Identifizierung und/oder Quantifizierung einer ersten Substanz, welche Kohlenhydrat bindet, das die folgenden Schritte umfaßt:
(a) Zugabe einer zu testenden Probe zu einem Trägermaterial, wobei das Trägermaterial einen immobilisierten Rezeptor trägt, welcher einen Teil der ersten Substanz spezifisch bindet, der nicht in die Kohlenhydratbindung involviert ist;
(b) Zugabe einer zweiten Substanz, die einen Kohlenhydrat-Anteil enthält, wobei der Kohlenhydrat-Anteil durch die erste Substanz in einem Komplex gebunden wird und wobei der Komplex nachweisbar ist; und
(c) Nachweis des Komplexes.

Der Begriff "Komplex" im Sinne dieser Erfindung umfaßt jeden chemischen Komplex zwischen der ersten Substanz und der zweiten Substanz, wie oben definiert. Nicht unter den begriff Komplex fallen damit kovalente Bindungen zwischen den genannten Substanzen.

Der Begriff "Nachweis des Komplexes" erfaßt erfindungsgemäß den Nachweis der Komplexbildung selbst, wie auch den Nachweis der zweiten Substanz im Komplex, wodurch wiederum der Komplex selbst nachgewiesen wird. Ausführungsformen, die unter die erste Alternative fallen, umfassen den Nachweis mit Antikörpern, die Strukturen spezifisch erkennen, die durch die Komplexbildung gebildet werden. Unter die zweite, in der Praxis bevorzugte Alternative fallen z.B. Nachweise mit Antikörpern, die die zweite Substanz spezifisch binden.

Der Begriff "spezifisch bindet" bedeutet im Sinne dieser Erfindung, daß der Rezeptor im wesentlichen monospezifisch ist und keine oder im wesentlichen keine Kreuzreaktivität insbesondere mit weiteren Substanzen mit Kohlenhydratbindeaktivität aufweist. Der Rezeptor ist vorzugsweise ein hochaffiner Rezeptor.

Der Begriff "Teil der ersten Substanz, der nicht in die Kohlenhydratbindung involviert ist" bedeutet erfindungsgemäß, daß dieser Teil weder in die Substratbindung selbst involviert und damit beispielsweise Teil eines aktiven Zentrums ist, noch, daß er die Substratbindung allosterisch beeinflußt. Der Fachmann kann auf der Basis der Lehre dieser Erfindung wie auch aufgrund seines allgemeinen Fachwissens ohne unzumutbaren Aufwand testen, ob der genannte Teil in die Kohlenhydratbindung involviert ist oder nicht. Beispielhaft wird nachstehend erläutert, wie der Fachmann anhand eines Mistellektin bindenden monoklonalen Antikörpers ermitteln kann, ob dieser an einen Teil der Mistellektinketten bindet, der in die Kohlenhydratbindung involviert ist. Zur Analyse dieser monoklonalen Antikörper können folgende Tests herangezogen werden:

### Test A:

An eine mit z.B. Asialofetuin (10 µg/Kavität) beschichtete Mikrotiterplatte wird mit biotinyliertem rML in einer Konzentrationsreihe inkubiert. Ein Nachweis des über die Kohlenhydratbindestellen an das Asialofetuin gebundene rML erfolgt über die Bindung von Streptavidin, welches wiederum mit HR-Peroxidase markiert ist, in einem kolorimetrischen Verfahren.

Zur Analyse der potentiellen Kompetition der Kohlenhydratbindung von monoklonalen Antikörpern werden diese zusammen mit dem rML koinkubiert. Die Roh-OD einer Probe ohne Zugabe von monoklonalem Antikörper im Vergleich zu der Probe mit zu testendem Antikörper sollte im Bereich der Meßgenauigkeiten gleich sein (+/-10%). Findet man einen Unterschied >10%, dann kann man davon ausgehen, daß der eingesetzte monoklonale Antikörper die Kohlenhydratbindung der rML B-Kette beeinflußt. Als interne Kontrolle (Glykosylierung des Antikörpers) wird ein beliebiger, kommerziell erhältlicher monoklonaler Antikörper aus der Maus, der nicht mit rML kreuzreagiert, in gleicher Konzentration eingesetzt. Inkubationszeiten und Temperaturen richten sich nach den in standardmäßig durchgeführten ELISA eingehaltenen Werten.

### Test B:

Eine weitere Möglichkeit zum Testen von monoklonalen Antikörpern könnte über die Bindung des rML an die Mikrotiterplatte (10 µg/ml) und die anschließende Bindung eines biotinylierten Asialofetuins an das rML und den Nachweis des gebundenen Asialofetuins mittels Streptavidin-Peroxidase erfolgen. Zur Analyse der potentiellen Beeinflussung der Kohlenhydratbindung durch die monoklonalen Antikörper werden diese zusammen mit dem biotinylierten Asialofetuin inkubiert. Auch hier würde ein Vergleich der Roh-OD einer ohne monoklonalen Antikörper inkubierten Probe mit einer mit monoklonalem Antikörper inkubierten Probe über eine Verwendbarkeit des Antikörpers gemäß den vorstehend diskutierten Kriterien entscheiden.

Mit dem erfindungsgemäßen Verfahren werden eine ganze Reihe von Nachteilen aus dem Stand der Technik beseitigt. Beispielsweise werden in den im Stand der Technik bekannten Verfahren Standardchargen bereitgestellt. Die daraus resultierenden Nachteile, z.B. Aktivität der Standardcharge, Qualität, Herstellerdifferenzen, fallen durch die Definition einer Volumenaktivität im erfindungsgemäßen Verfahren weg.

Mit dem Erreichen einer systemabhängigen Bezugsgröße in Form einer Sättigung der Absorption bei 490 nm, kann nämlich die Bestimmung eines IC₅₀-Wertes (Wendepunkt der sigmoiden Kurve) als Maß des Aktivitätsgehaltes einer unbekannten Probe etabliert werden. Eine systeminterne Bezugsgröße in Form einer Sättigung der Bindeplätze konnte für das Mistellektin im "konventionellen" ELLA trotz zahlreicher Variationen und Optimierungen (pH-Wert, Inkubationsdauer und -temperatur) nicht erreicht werden, da die Gleichgewichtsdissoziationskonstanten (K_{D}) von Lektinen zu ihren Kohlenhydrat-Liganden im µM-Bereich liegen und damit eine relativ schwache Wechselwirkung beschreiben. So finden Shimoda & Funatsu (1985) bei dem ML-Homolog Ricin für Lactose einen K_{D}-Wert von 43 µM und für Galactose einen K_{D}-Wert von 164 µM, Yamashita et al. (1992) bestimmen für ein Lektin aus Wurzeln von *Trichosanthes japonica* einen KD-Wert von 1.3 µM. Gabius et al. (1992) bestimmten für die Wechselwirkung von ML an THP-1 Zellen einen K_{D}-Wert von 0.8 µM.

Erfindungsgemäß wird durch das Verlagern der starken Wechselwirkung zwischen immobilisiertem Rezeptor, z.B. einem Fangantikörper, und Mistellektin (K_{D}-Werte im 10⁻⁹ M Bereich) an den Beginn der Detektionskaskade und die Bindung der zweiten Substanz, z.B. des glycosylierten Asialofetuins, an das zu analysierende Kohlenhydratbindende Protein (z.B. ML) im letzten Schrill (K_{D}-Werte im 10⁻⁶ M-Bereich) unmittelbar vor die eigentliche Detektionsreaktion vorteilhafterweise erreicht, daß während der Inkubationsphasen wie auch während der Waschphasen der belegten Mikrotiterplatte weniger Diffusionsereignisse zu erwarten sind.

Das erfindungsgemäße Verfahren ist gegenüber dem erwähnten Stand der Technik weiterhin von Vorteil, da Proben (in einer bevorzugten Ausführungsform Mistellektin enthaltende Proben) in Anwesenheit von kompetitierenden Kohlenhydraten vermessen werden können, da die Kohlenhydrat-Aktivität erst nach Bindung des Kohlenhydrat-bindenden ML-Moleküls über die Rezeptor-Ketten-Wechselwirkung an die Platte erfolgt.

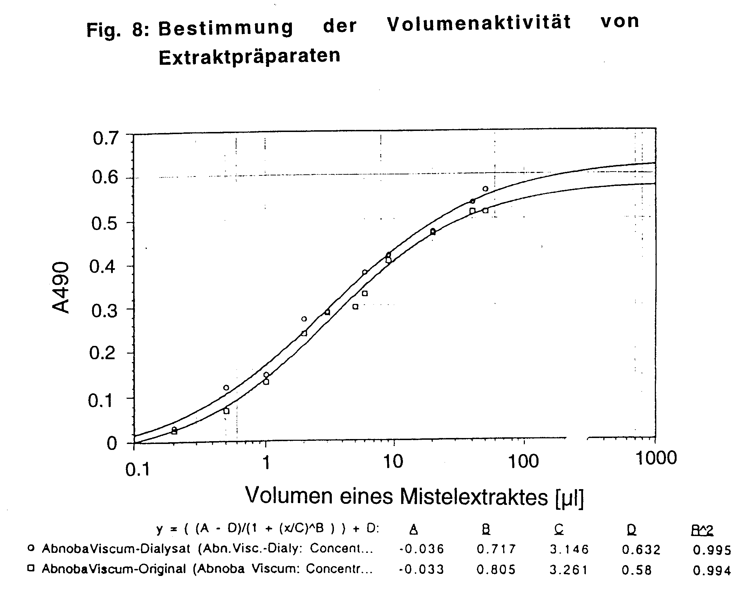

Mit dem erfindungsgemäßen Verfahren ist man auch in der Lage, marktübliche Extraktpräparate zu vermessen. In Beispiel 7 wird die Vermessung von heterogenen Extraktpräparaten gezeigt. Durch die Anwendung des ILA konnte ein negativer Einfluß von eventuell störenden Oligosacchariden oder kompetitiven Kohlenhydraten ausgeschlossen werden, was durch Vermessen eines Extraktpräparates im Herstellerpuffer im Vergleich zu einer auf PVP-Puffer (20 mM Phosphat, pH 8.0, 300 mM NaCl, 1 mM EDTA, 100 µg/ml PVP) dialysierten Probe bestätigt werden konnte (Fig. 8).

Durch den Einsatz eines monoklonalen Antikörpers gemäß einer besonders bevorzugten Ausführungsform, der nur gegen einen nicht in die Kohlenhydratbindung involvierten Teil der Substanz, z.B. die ML-A-Kette, gerichtet ist, können zudem Kreuzreaktionen minimiert werden. Der Test hat daher in der Praxis eine höhere Spezifität gegenüber ML gezeigt.

Erfindungsgemäß wird im Gegensatz zu den bisher beschriebenen Anordnungen zur Durchführung der Bestimmungsverfahren zu Beginn der Detektionskaskade die sehr spezifische Bindung des Lektins über einen die Kohlenhydrat-Bindung nicht beeinflussenden Rezeptor, vorzugsweise monoklonalen Antikörper gelegt. Aufgrund der Rezeptorbeschichtung/Antikörperbeschichtung mit nachgeschalteter Lektin-Zugabe in den ersten beiden Schritten konnte die gegen störende Pufferkomponenten sensible Lektin-Kohlenhydrat-Erkennung im Aufbau zeitlich an das Ende der Detektionskaskade verschoben werden. Die Erkennung des an den Antikörper gebundenen Lektins erfolgt dann beispielsweise mit einer Komplexlösung, bestehend aus dem markierten Kohlenhydrat-tragenden Molekül (z.B. biotinyliertes Asialofetuin) und ein Detektionsreagenz (z.B. Avidin-Peroxidase). Eine Messung der nachweisbaren Markierung erfolgt über die Ermittlung der Aktivität (z.B. des Enzymumsatzes), vorzugsweise in einem colorimetrischen Verfahren.
Zusammenfassend läßt sich feststellen, daß es mit dem erfindungsgemäßen Verfahren somit gelungen ist, einen systeminternen Bezugspunkt zu erreichen, der eine Standardunabhängigkeit ermöglicht. Es wurde eine Kohlenhydrat-Bindeaktivität und in einer bevorzugten Ausführungsform eine Lektin-Bindeaktivität und in einer besonders bevorzugten Ausführungsform Mistellektin-Bindeaktivität definiert, die in Form einer Aktivitätseinheit beschrieben werden kann. Dabei kann die Aktivität auch in solchen Lösungen bestimmt werden, die in bisherigen Meßsystemen falsche, insbesondere zu geringe ("falsch negative"), Signale geliefert hätten. Weiterhin gelang es, durch die Einführung einer systeminternen Bezugsgröße, verschiedene Mistellektin-Proben Standardchargen-unabhängig zu vermessen. Der ILA ist damit von großem Interesse für Hersteller standardisierter ML-Präparate.

In einer bevorzugten Ausführungsform ist die erste Substanz ein Lektin.
Laut einer Einteilung von Molekülen mit Kohlenhydrat-Bindeaktivität in vier Lektin-Klassen (Sharon, 1993), die mit dem erfindungsgemäßen Verfahren erfaßbar sind, gibt es eine Reihe von Kohlenhydrat-bindenden Molekülen, die man aufgrund ihrer Aminosäure-Primärstuktur nicht in eine dieser Klassen einordnen kann, wie z.B. das Cytokin Interleukin-2 (IL-2) (Zanetta et al., 1996) oder das Mannose-Bindeprotein aus Dendritischen Zellen oder Makrophagen (Stahl & Ezekowitz 1998). Diese Kohlenhydrat-bindenden Moleküle werden durch andere bevorzugte Ausführungsformen des erfindungsgemäßen Verfahrens ebenso indentifiziert/quantifiziert, wie monoklonale Antikörper, funktionelle Fragemente oder funktionelle Derivate davon, die eine spezifische Glycosylierung (z.B. an einem Protein oder einem Glycolipid) erkennen.

Besonders bevorzugt ist, daß die erste Substanz ein pflanzliches Lektin ist.

In einer weiteren besonders bevorzugten Ausführungsform ist das pflanzliche Lektin ein Mistellektin.

In einer weiteren bevorzugten Ausführungsform ist die zweite Substanz, die einen Kohlenhydrat-Anteil enthält, ein Glykoprotein, vorzugsweise Asialofetuin, ein Glykolipid, ein Kohlenhydrat oder ein synthetisches Substrat mit Kohlenhydrat-Anteil oder ein Neoglycoprotein, vorzugsweise Galactosyl-Rinderserumalbumin.

In einer zusätzlichen bevorzugten Ausführungsform ist das synthetische Substrat mit Kohlenhydratanteil ein Neoglycoprotein, vorzugsweise Poly-N-Hydroxyethyl-N-SP-Biotinyl-N-SP-Glycosyl-Acrylamid.

In einer weiteren bevorzugten Ausführungsform ist der immobilisierte Rezeptor ein Antikörper, ein Aptamer oder ein Enzym.
Unter den Begriff "Antikörper" fallen erfindungsgemäß auch Fragmente oder Derivate von Antikörpern, die natürlichen Ursprungs, synthetischer oder semisynthetischer Natur sein können. Fragmente umfassen Fab- oder F(ab')₂ ―Fragmente. Die Herstellung von Antikörpern und Fragmenten davon ist beispielsweise beschrieben in Harlow und Lane (1988). Derivate schließen scFv-Fragmente ein sowie Derivate, die durch Peptidomimetics hergestellt wurden. Letzteres gilt auch für die im erfindungsgemäßen Verfahren eingesetzten Enzyme. "Aptamere" und ihre Herstellung sind im Stand der Technik beschrieben beispielsweise in Osborne et al. (1997), sowie in Stull und Szoka (1995).

Besonders bevorzugt ist, daß der Antikörper ein monoklonaler Antikörper ist.

Weiterhin ist bevorzugt, daß die zweite Substanz, welche einen Kohlenhydrat-Anteil enthält, nachweisbar markiert ist. In dieser bevorzugten Ausführungsform ist besonders bevorzugt, daß die Markierung mit der zweiten Substanz kovalent verbunden ist.

Ferner ist besonders bevorzugt, daß die Markierung eine dritte Substanz ist, die eine vierte Substanz spezifisch bindet.

In einer weiteren bevorzugten Ausführungsform erfolgt der Nachweis vermittels eines zweiten Rezeptors, welcher spezifisch an die zweite Substanz bindet. Die Rezeptorbindung ist üblicherweise eine Komplexbildung, die unter z.B. physiologischen Bedingungen ablaufen kann.

Besonders bevorzugt ist, daß der zweite Rezeptor nachweisbar markiert ist.

In einer weiteren bevorzugten Ausführungsform der erfindungsgemäßen Verfahrens setzt man für den Nachweise einen dritten Rezeptor zu, wobei der zweite Rezeptor durch einen dritten Rezeptor gebunden wird und wobei der dritte Rezeptor, vorzugsweise durch ein daran gekoppeltes Enzym, nachweisbar markiert ist.

In einer weiteren besonders bevorzugten Ausführungsform ist die dritte Substanz Biotin, Avidin oder Streptavidin.

Weiterhin ist besonders bevorzugt, daß die vierte Substanz Biotin, Avidin oder Streptavidin ist.
Die beiden letztgenannten Ausführungsformen sind insofern funktionell miteinander verknüpft, als die dritte Substanz mit der vierten Substanz ein bindendes Paar ausbilden soll. Ist beispielsweise die dritte Substanz Biotin, so sollte die vierte Substanz Avidin oder Streptavidin sein. Ist andererseits die dritte Substanz Avidin oder Streptavidin, so sollte die vierte Substanz Biotin sein.

In einer anderen besonders bevorzugten Ausführungsform ist der zweite und/oder dritte Rezeptor ein Antikörper, ein Aptamer oder ein Enzym.
Hinsichtlich der Definitionen für Antikörper und Aptamere wird auf die vorstehende Beschreibung verwiesen.

Weiterhin ist bevorzugt, daß die vierte Substanz nachweisbar markiert ist.

Besonders ist dabei auch bevorzugt, daß die Markierung ein an die vierte Substanz gekoppeltes Enzym gekoppelt ist.

In einer anderen besonders bevorzugten Ausführungsform ist die Markierung eine fluoreszierende, chemilumineszierende, biolumineszierende oder radioaktive Markierung oder colloidales Gold.

In einer weiteren besonders bevorzugten Ausführungsform erfolgt der Nachweis durch Spektrometrie, Fluorimetrie, Luminometrie, Radiometrie, Potentiometrie oder enzymatisch / katalytisch.

Weiterhin ist bevorzugt, daß das Enzym durch eine colorimetrische Substratumsetzung nachgewiesen wird, vgl. Harlow und Lane, a.a.O.

Besonders bevorzugt ist, daß das Enzym Meerrettich-Peroxidase oder alkalische Phosphatase ist.
Die im ILA einzusetzenden Substrate sind zum einen 5-Brom-4-chlor-3-indolyl Phosphat (#B0274) für die alkalische Phosphatase-Nachweisreaktion sowie o-Phenylendiamin (#P8287) für die Peroxidase-Nachweisreaktion. Die Substrate sind bei Sigma unter den genannten Nummern erhältlich.

Auch ist bevorzugt, daß die zweite Substanz zusammen mit den weiteren Substanzen und/oder Rezeptoren in einem Schritt zugegeben wird.
Die gleichzeitige Zugabe beispielsweise des biotinylierten Asialofetuins und des Nachweissystems bestehend aus z.B. Avidin-Peroxidase reduziert die Zahl der Diffusionsereignisse während der Inkubation und erleichtert dadurch das Erreichen der systeminternen Größe der Sättigung.

In einer besonders bevorzugten Ausführungsform wird die erste Substanz in einem Rohextrakt nachgewiesen und/oder quantifiziert.
Eine solche Ausführungsform bietet die Gelegenheit als heterogene Lösungen auf dem Markt befindliche Extraktpräparate trotz eines möglichen Gehalts an kompetitiven Kohlehydraten bezogen auf den Gehalt an Wirkstoff (z.B. Mistellektin) zu qantifizieren und ggf. zu normieren.

In einer weiteren besonderen Ausführungsform wird eine Sättigung der Bindung der ersten Substanz unter einer Konzentration von 50 µg/ml, bevorzugt unter einer Konzentration von 5 µg/ml, stärker bevorzugt unter einer Konzentration von 2 µg/ml und besonders bevorzugt unter einer Konzentration von 0,9 µg/ml erreicht.
Die Sättigung stellt im erfindungsgemäßen Verfahren ein wesentliches Ereignis dar. Das Erreichen der Sättigung ist jedoch abhängig von der Spezifität und der Konzentration an immobilisiertem Rezeptor.
Mit dem hier beschriebenen Verfahren wird eine Lösung der Nachteile vom Stand der Technik aufgezeigt. Bisher ist es diversen Autoren nicht gelungen, die systeminterne Sättigung bei niedrigen Analyt-Konzentrationen zu erreichen, was hier aufgrund der Invertierung des Testaufbaus im Vergleich zu dem im Stand der Technik bekannten Testaufbau gelungen ist.

Auch ist bevorzugt, daß das feste Trägermaterial eine Mikrotiterplatte, ein Teströhrchen oder ein Kügelchen (Bead) ist.

Darüber hinaus betrifft die Erfindung ein Test-Kit zur Ausführung des erfindungsgemäßen Verfahrens, enthaltend:
(a) einen Rezeptor, wie vorstehend definiert, welcher einen Teil der ersten Substanz spezifisch bindet, der nicht in die Kohlenhydratbindung involviert ist;
(b) eine zweite Substanz, wie vorstehend definiert; und
(c) eine Detektionssystem zum Nachweis der zweiten Substanz.

Es ist weiterhin bevorzugt, daß in dem Test-Kit
(a) der Rezeptor, welcher einen Teil der ersten Substanz spezifisch bindet, der nicht in die Kohlenhydratbindung involviert ist, ein monoklonaler Antikörper ist;
(b) die zweite Substanz biotinyliertes Asialofetuin ist, und
(c) das Detektionssystem mit Meerrettich-Peroxidase konjugiertes Avidin ist.

Besonders bevorzugte Anwendungsmodi dieses Test-Kits sind nachfolgend beschrieben.

Die Komponenten des erfindungsgemäßen Kits können getrennt oder zusammen verpackt/konfektioniert sein, wobei die getrennte Verpackung der Komponenten bevorzugt ist.

Weiterhin ist besonders bevorzugt, daß in einem Test-Kit der Rezeptor an ein Trägermaterial immobilisiert ist. Das Trägermaterial ist vorzugsweise wie vorstehend dargestellt ausgestaltet.

Nachfolgend werden einige Beispiele bzw. bevorzugte Ausführungsformen des erfindungsgemäßen Verfahrens zum besseren Verständnis der Erfindung im Zusammenhang dargestellt.

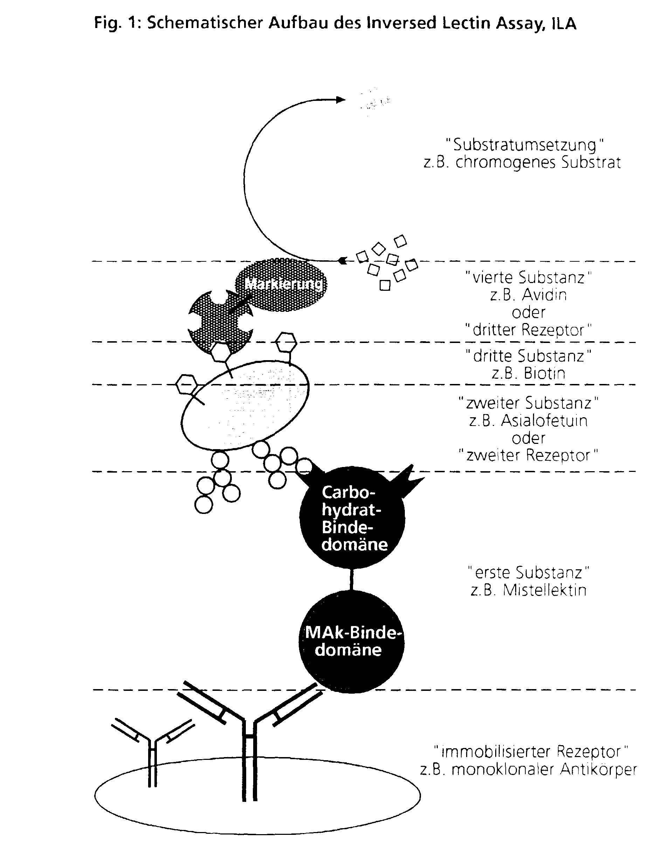

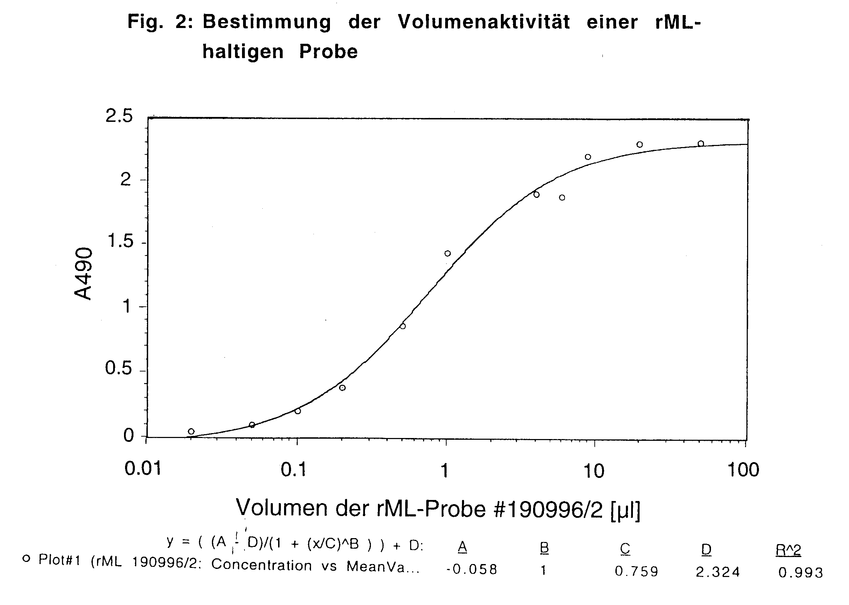

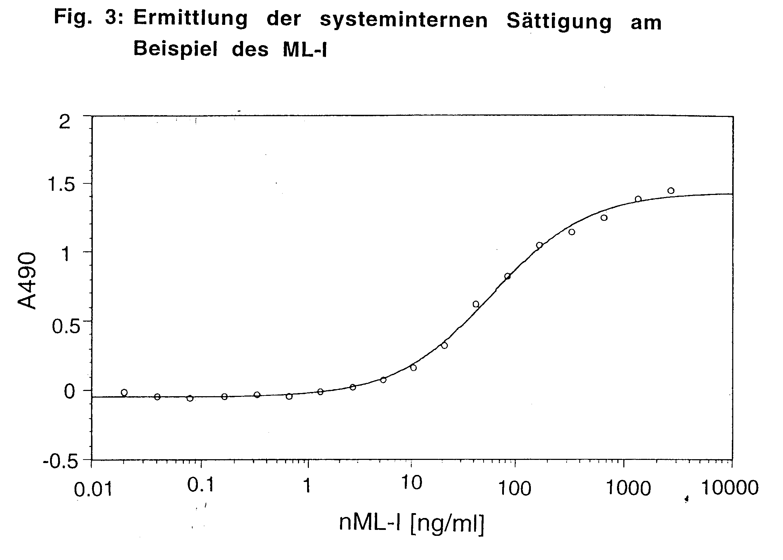

Im ersten Schritt des ILA erfolgt eine Beschichtung des Trägermaterials, vorzugsweise eine Mikrotiterplatte, mit dem die Kohlenhydrat-Bindung nicht beeinflussenden hochaffinen Rezeptor. In einer bevorzugten Ausführungsform handelt es sich hier um einen spezifischen monoklonalen Antikörper. In der Ausführungsform zum Nachweis von ML wird ein monoklonale Antikörper gegen die A-Kette (z.B. TA5) eingesetzt. Dieser Antikörper wurde auf konventionellem Wege hergestellt; vgl. Harlow und Lane (1988). Aufgrund der hohen Affinität des Antikörpers zu ML-I (niedriger K_{D}-Wert im Bereich von 10⁻⁹ M; Tonevitzky et al., 1995), gelingt es, die an die Platte gebundenen Antikörper quantitativ mit dem zu detektierenden bzw. zu quantifizierenden Lektin (in diesem Fall ML) zu binden. Die weniger affine Wechselwirkung zwischen dem Lektin und der zweiten Substanz, die den Karbohydrat Anteil trägt und vorzugsweise ein Glykoprotein, im Falle des ML Asialofetuin, ist, wurde in diesem Aufbau an das Ende der Detektionskaskade gesetzt (Abbildung 1: schematischer Aufbau). Neben dem Karbohydrat-Anteil trägt die zweite Substanz noch eine zusätzliche Markierung. Diese Markierung kann entweder direkt nachweisbar sein (z.B. Fluoreszenz, Radioaktivität, Goldmarkierung) oder in einer bevorzugten Ausführungsform eine dritte Substanz sein, die über eine Wechselbeziehung mit einer vierten Substanz erkannt wird. In einer besonders bevorzugten Ausführungsform ist die dritte Substanz Biotin und die vierte Substanz Avidin-Peroxidase oder Streptavidin-Peroxidase.
Abhängig von dem Gehalt an und der Bindeaktivität von ML in einer Probe wird eine Dosis-Signal-Kurve über die Umsetzung eines Substrats (z.B. colorimetrisch) aufgenommen, die durch den Einsatz steigender Volumina in einer systeminternen Sättigung endet. Mit dem systeminternen Bezugspunkt der "Sättigung" wird über die Ermittlung der halbmaximalen Sättigung (IC₅₀-Wert; Vier-Parameter-Fit) eine Volumenaktivitätseinheit (U/ml) als Maß des Gehaltes sowie der Aktivität an Kohlenhydratbindendem Material definiert. Durch den Bezug auf die Gesamtproteinkonzentration mittels Proteintest (z.B. Bradford-Assay) wird zudem die Berechnung einer spezifischen Aktivität (U/µg) ermöglicht.
Mit einer Variation des Volumens von z.B. einer rekombinant dargestellten ML Präparation (rML) unbekannter Konzentration kann ein Volumenbereich gefunden werden, der einen sigmoiden Verlauf beschreibt (Beispiel 1, Fig.2). In Abhängigkeit des Gehaltes an rML in einer Charge variiert das Volumen, welches zum Erreichen der halbmaximalen Sättigung benötigt wird. Je kleiner das benötigte Volumen ist (IC₅₀), desto höher ist die Volumenaktivität einer Probe. Ebenso konnte in einer weiteren besonders bevorzugten Ausführungsform mit dem natürlichen Mistellektin I (ML-I) dosisabhängig eine Sättigung erreicht werden (Beispiel 2, Fig. 3).

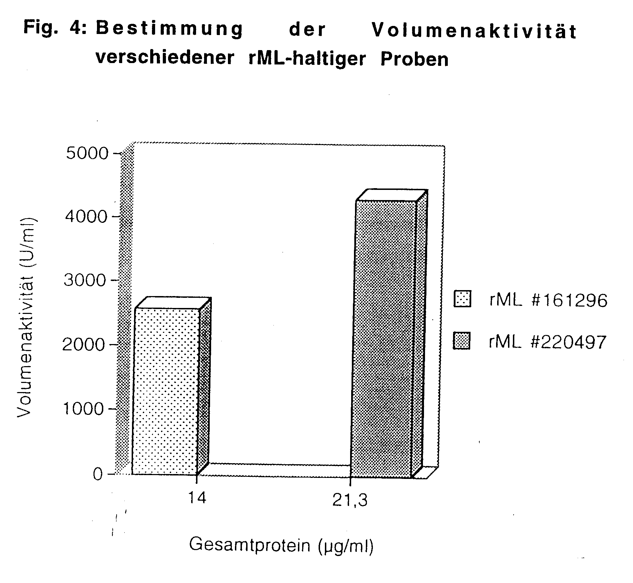

In Beispiel 3 wurden zwei verschiedene rML-Chargen mittels ILA auf ihre Volumenaktivität untersucht. Hierbei konnten zwei unterschiedliche Volumenaktivitäten beschrieben werden, die sich um den Faktor 1.6 unterschieden (Fig. 4). Der im ILA ermittelte Faktor konnte auch in Mikro-Bradford bestätigt werden, was letztlich zeigte, daß die spezifischen Aktivitäten (U/µg) für die über den gleichen Prozeß gereinigten Proteine praktisch identisch waren (Tabelle 1). Eine Beschreibung von verschiedenen Lektin-enthaltenden Proben, vorzugsweise Mistellektin enthaltenden Proben, erfolgt hier nicht mehr über die Bezeichnung "Äquivalente der Standardcharge" sondern über eine Substanzeigenschaft der Kohlenhydrat-bindenden Proteine.

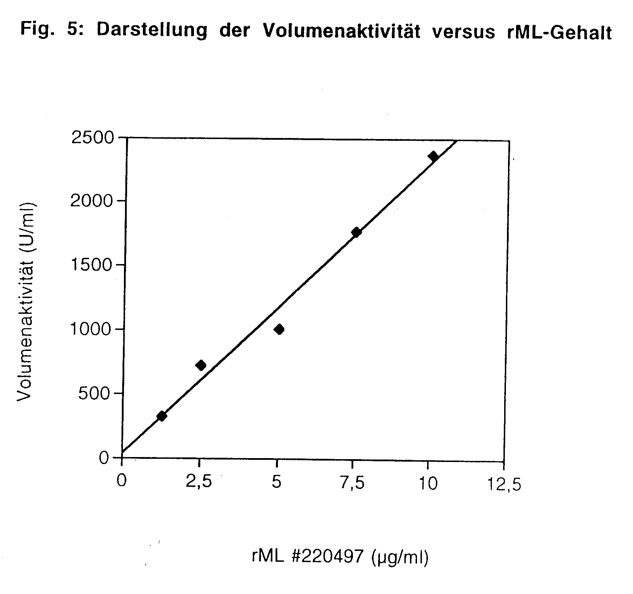

In Beispiel 4 ist die Richtigkeit bzw. die Plausibilität ( 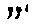 accuracy") des Tests beschrieben (Fig. 5). Hier wurde eine rML-Charge seriell 1:2 verdünnt. Die verschiedenen Verdünnungen wurden im Anschluß im ILA vermessen. Im Konzentrationsbereich von 1 µg/ml bis 10 µg/ml konnte dabei eine linearere Korrelation zwischen Volumenaktivität und Gehalt konform der jeweiligen Verdünnung beobachtet werden. Der oben erwähnte Konzentrationsbereich stellt gleichzeitig den Bestimmungsbereich des ILA-Tests dar.

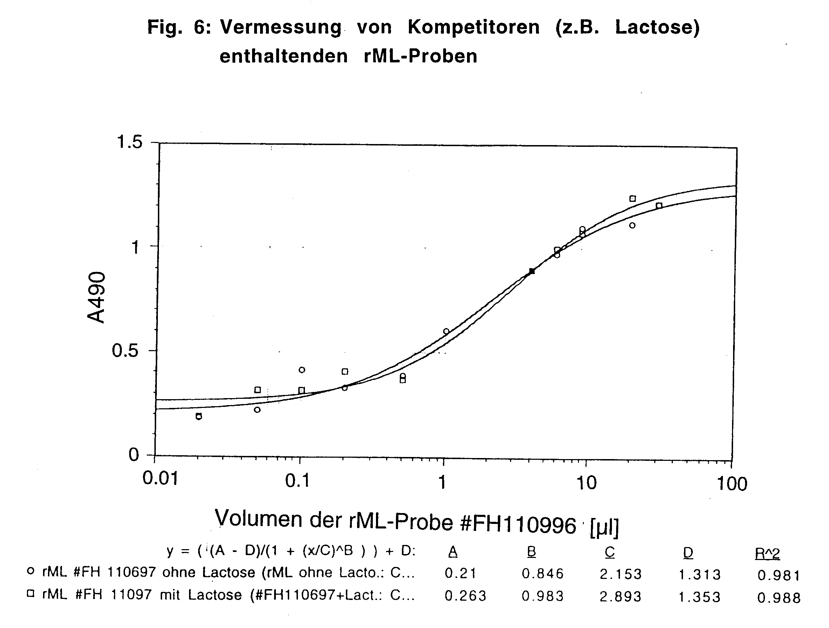

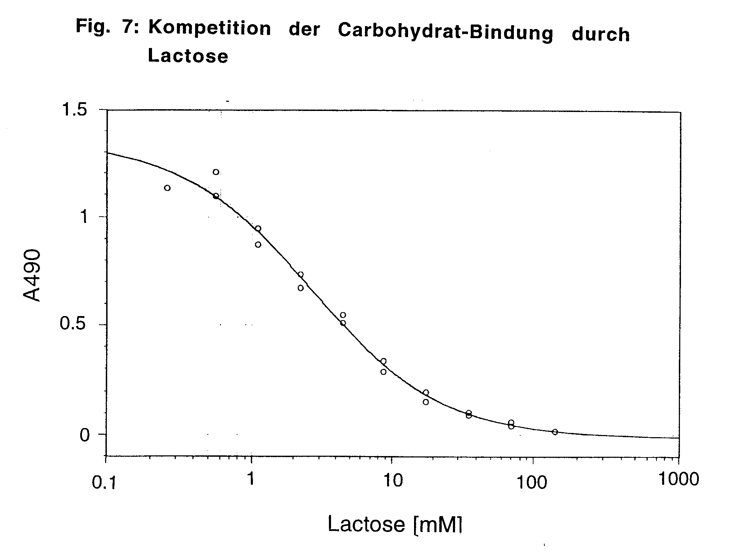

In Beispiel 5 wurde rML in steigenden Volumina auf einer mit TA5 beschichteten Platte inkubiert. Hierbei wurden zum einen Proben ohne Zugabe des kompetitiven Kohlenhydrates Lactose (20 mM) als Kontrolle und zum Vergleich rML-Proben zeitgleich mit 20 mM Lactose inkubiert (Fig. 6). Nach der Inkubation der Proben an der TA5 Matrix wurde die Platte gewaschen. Dadurch konnten die Folgebedingungen (spez. Wechselwirkung der B-Kette mit Asialofetuin) in einem für diese Reaktion optimierten Puffersystem erfolgen. Vergleicht man die Volumenaktivitäten dieser beiden Ansätze, so stellt man fest, daß bei der Inkubation des immobilisierten Rezeptors, beispielsweise des monoklonalen Antikörpers TA5, mit dem Lektin, besonders bevorzugt Mistellektin, die kohlenhydrat-Wechselwirkung kompetitierende Agenzien, wie z.B. Lactose, keinen signifikanten Einfluß auf die Volumenaktivität haben.
Eine Kompetition der Kohlenhydrat-Wechselwirkung zwischen der B-Kette und dem Asialofetuin (Beispiel 6) konnte mit Lactose beschrieben werden. Hier wurde ein definiertes Volumen rML über den mAk TA5 gebunden. Bei der anschließenden Inkubation des Asialofetuin-Sandwiches wurde Lactose in einem Konzentrationsbereich von 280 µM bis 140 mM eingesetzt (Fig. 7). Die Hälfte der gebundenen rML-Moleküle konnten mit 2.6 mM Lactose verdrängt werden (IC₅₀).

Die Figuren zeigen:
Fig. 1: Schematischer Aufbau einiger Ausführungsformen des Inversen Lectin-Assay (ILA)
Fig. 2: Bestimmung der Volumenaktivität einer rML-haltigen Probe
Fig. 3: Ermittlung der systeminternen Sättigung am Beispiel des ML-I
Fig. 4: Bestimmung der Volumenaktivität verschiedener rML-haltiger Proben
Fig. 5: Darstellung der Volumenaktivität versus rML-Gehaltes
Fig. 6: Vermessung von Kompetitoren (z.B. Lactose) enthaltenden rML-Proben
Fig. 7: Kompetition der Kohlenhydrat-Bindung durch Lactose
Fig. 8: Bestimmung der Volumenaktivität von Extraktpräparaten

Die Beispiele erläutern die Erfindung.

### Beispiel 1: Allgemeiner Aufbau des ILA Tests zur Volumenaktivitätsbestimmung von Kohlenhydrat-bindenden Molekülen

Im ersten Schritt des ILA-Sandwich-Tests erfolgt eine Beschichtung der 96-Loch-Platte (Nunc Immuno Platte #446612) ü.N. mit dem die Mistellektin A-Kette erkennenden monoklonalen Antikörper (mAk) TA5. Die Konzentration des mAks beträgt 3 µg/ml in PBS (50 mM Na₂HPO4, pH 7.4, 130 mM NaCl). Pro Kavität werden 100 µl der Lösung pipettiert und über Nacht bei RT inkubiert. Nach 3x Waschen der Platte mit PBS-T (50 mM Na₂HPO4, pH 7.4, 130 mM NaCl, 0.05 % Tween 20) wird die Platte 1 h bei RT mit je 200 µl PBS 1% (w/v) BSA (50 mM Na₂HPO4, pH 7.4, 130 mM NaCl + 1% (w/v) BSA von Sigma #A3803) geblockt. Nach erneutem 3x Waschen mit 150 µl PBS-T wird an den Antikörper Mistellektin über die A-Kette gebunden (Inkubation 1 h, bei 37 °C). Hier werden routinemäßig die Volumina 30/20/9/6/4/1/0.5/0.2/0.1/0.05/0.02/0 µl pipettiert, wobei für die Endvolumina 4 µl und 1 µl in die Platte 40 µl bzw. 10 µl einer 1:10 Verdünnung in PVP-Puffer (20 mM Na₂HPO4, pH 8.0, 300 mM NaCl, 1 mM EDTA, 100 µg/ml Polyvinylpyrolidon-10, (Sigma #P6755) und für die Volumina 0.5, 0.2, 0.1 0.05 sowie 0.02 ausgehend von einer 1:100 Verdünnung der Ausgangslösung in PVP-Puffer 50, 20, 10, 5 und 2 µl pipettiert werden. Die verschiedenen Volumina werden ad 50 µl auf PVP-Puffer und ad 100 µl auf PBS 0.05% BSA gebracht. Pro Meßwert werden drei individuelle Kavitäten pipettiert, aus denen nach Vermessen der Platte Mittelwerte gebildet werden. Im folgenden schließen sich nach 3x Waschen mit 150 µl PBS 0.05% BSA zwei Reaktionen gekoppelt in einem Schritt an. 100 µl Biotinyliertes Asialofetuin, in einer Konzentration von 400 µg/ml in PBS+ 0.05% (w/v) BSA (Herstellung: Biotinylierungsagens von Pierce (#21335) und Asialofetuin (Typ I, Fetal Calf Serum, Sigma #A4781) werden im molaren Verhältnis 1:20 laut Protokollbuch von Pierce inkubiert und dialysiert) wird zusammen mit 100 µl einer 1:75 Vorverdünnung der Avidin-Peroxidase (Sigma #A7419) in PBS 0.05% BSA (w/v) in 9.8 ml des der B-Ketten-Kohlenhydrat-Bindeaktivität angepaßten Acetat- Puffers (50 mM Na-Acetat, pH 5.6, 100 mM NaCl, 1 mM EDTA, 0.05% (w/v) BSA) aufgenommen. Es erfolgt parallel die Bindung des Asialofetuins an die B-Kette und die Wechselwirkung des Biotins mit dem Avidin. Bei der abschließenden Substratreaktion werden eine OPD-Tablette (Sigma #P8287) in 25 ml 50 mM Na-Citrat-Puffer, pH 5.0 (vortemperiert auf 25 °C) gelöst. Kurz vor Zugabe von je 100 µl Substratlösung wird der 25 ml Substratlösung 10 µl H₂O₂ zugegeben und gut verrührt. Nach 30 min Inkubation bei 25 °C wird die Reaktion durch Zugabe von 100 µl 1 M H₂SO₄ abgesoppt. Die Platte wird in einem Mikroplatten Reader bei 490 nm vermessen. Die Pufferblanks wie auch die A690 nm werden von den Rohdaten subtrahiert. Es erfolgt eine Auftragung der Absorption bei 490 nm gegen die eingesetzten Volumina der ML-Probe. Abhängig von der Aktivität der ML-Lösung kommt es zum Anstieg der A490 in Abhängigkeit des eingesetzten Volumens bis hin zum Ausbilden einer Sättigung. Eine Sättigung gilt als erreicht, wenn die A490 der letzten drei Volumina (9, 20 und 30 µl) +/- 10% gleich sind. Mit Hilfe des Vier-Parameter-Fits (Software: Softmax PRO von Molecular Devices) kann nun der Wendepunkt der sigmoiden Kurve ermittelt werden. Dieser Wendepunkt beschreibt ein definiertes Volumen und wird als eine Volumeneinheit definiert. Der Wert (in µl) der Volumeneinheit ist umso größer je weniger rML in einer Lösung vorhanden ist. Der Bezug auf ein Milliliter (1000 µl / Wert C) führt dann zur Volumenaktivität (U / ml). Für die in Fig. 2 dargestellte rML-Charge ergibt sich somit eine Volumenaktivität von 1 U/0.759 µl oder 1318 U/ml.

### Beispiel 2: Ermittlung der systeminternen Sättigung am Beispiel des ML-I

Ausgehend von einer ML-I Standardlösung (#220793) wurde die ML-Konzentration ermittelt, bei der das System die Sättigung erreicht.
Mistellektin I (ML-I) wurde in einem Konzentrationsbereich von 0.02 ng bis 2800 ng/ml eingesetzt. Ausgehend von einer 100 µg/ml Stammlösung in PVP-Puffer wurden 1:10, 1:100 und 1:1000 Zwischenverdünnungen in PVP-Puffer hergestellt. Die der jeweiligen ML-I Quantität entsprechenden Volumina wurden ad 50 µl PVP-Puffer pipettiert und ad 100 µl mit PBS + 0.05% (w/v) BSA ergänzt. Der grundsätzliche Verlauf des ILA erfolgte wie in Beispiel 1 beschrieben. Es kann eine deutliche Abhängigkeit der Absorption bei 490 nm (A490) von der eingesetzten Quantität an ML-I beobachtet werden (Fig. 3). Zu Beginn (0.02 ng/ml bis 10 ng/ml) zeigt sich eine schwache Abhängigkeit der A490 von der ML-Zugabe schließt sich eine lineare Abhängigkeit der A490 von der ML-Zugabe an (10 -700 ng/ml), die dann bei einer ML-I Konzentration von etwa 900 ng/ml in eine Sättigung der A490 übergeht.

### Beispiel 3: Ermittlung von Volumenaktivitäten unterschiedlicher rekombinanter Mistellektin Proben

Das experimentelle Vorgehen entsprach dem in Beispiel 1 dargestellten.
Fig. 4 zeigt ein Balkendiagramm, bei dem die Volumenaktivität zweier Chargen gegen den Proteingehalt laut Mikro-Bradford dargestellt ist. Eine Zusammenfassung der Ergebnisse sind in der unten stehenden Tabelle 1 zusammengefaßt.

**Tabelle 1**

| Ermittlung von spezifischen Aktivitäten zweier rML-Proben | | | |
|---|---|---|---|
| rML-Probe | Volumenaktivität laut ILA (U/ml) | Gesamtprotein laut Mikro-Bradf. (µg/ml) | spezifische Aktivität (U/µg) |
| #161296 | 2573 | 14 | 184 |
| #220497 | 4310 | 21.3 | 202 |

Die Tabelle zeigt, daß sich die beiden aufgeführten Proben, die über den identischen Herstell- und Reinigungsprozeß dargestellt wurden, sich zwar in ihrer Volumenaktivität (U/ml) unterschieden, doch über den Bezug mit einem Gesamtprotein-Test (Bradford) eine nahezu Identische spezifische Aktivität (U/µg) aufweisen.

### Beispiel 4: Richtigkeit des ILA-Tests

Eine rekombinante Mistellektin-Probe wurde mittels ELLA-Quantifizierungs-Test quantifiziert. Es wurden zwei Stammlösungen von 15 µg/ml und 10 µg/ml in PVP-Puffer angelegt, die im folgenden seriell verdünnt wurden.
Von den jeweiligen Verdünnungen (1.25 µg/ml, 2.5 µg/ml, 5 µg/ml, 7.5 µg/ml, 10 µg/ml) wurden immer gleiche Volumina im ILA eingesetzt (30 µl bis 0,02 µl). Die Auswertung erfolgte nach Auftragung der A490 gegen die steigenden Volumina mittels Vier-Paramerter-Fit. Die so ermittelten Volumenaktivitäten wurden gegen die steigende rML-Konzentration aufgetragen (Fig. 5). Es zeigt sich ein linearer Zusammenhang zwischen der Volumenaktivität und dem rML-Gehalt einer Probe. In dem getesteten Konzentrationsbereich führt eine Verdopplung der rML-Konzentration auch zu einer Verdopplung der Volumenaktivität.

### Beispiel 5: Bestimmung der Volumenaktivität in Anwesenheit kompetitierender Karbohydrate

Rekombinantes Mistellektin #FH110697 wurde in steigenden Volumina (0/0.02/0.05/0.1/0.2/0.5/1/4/6/9/20/30 µl) an eine mit dem mAK TA5 beschichtete Platte gebunden. Bei diesem Einfangvorgang wurde einer Meßreihe zusätzlich zu den Pufferkomponenten 20 mM Lactose zugegeben (Fig. 6), eine Konzentration, die die Aktivität der B-Kette quantitativ kompetitieren würde (siehe Fig. 7). Der Einfluß des kompetitiven Karbohydrates Lactose bei der Bindung des rML an den TA5 Fangantikörper auf die Volumenaktivität (VA) war nicht signifikant (VA + Lactose: 1 Unit = 2.89 µl; entspr.: 345 U/ml; VA -Lactose: 1 Unit = 2.15 µl;entspr.: 465 U/ml).

### Beispiel 6: Kompetition der Kohlenhydrat-Bindung durch Lactose

Ein definiertes Volumen rML (# 161296) entsprechend 100 ng rML in der Kavität (Bestimmung laut ELLA) wurde über den TA5 Antikörper an die 96-Loch-Platte gebunden. Der folgende Schritt der Bindung des biotinylierten Asialofetuins an die B-Kette des rML-Moleküls wurde unter steigenden Konzentrationen des kompetitiven Kohlenhydrates β-D-Lactose (0.28 bis 140 mM) durchgeführt (Fig. 7). Eine Auswertung der Kurve ergab, daß zur Kompetition von 50% des Asialofetuins 2.6 mM Lactose ausreicht. Bereits bei einer Konzentration von 20 mM Lactose sind >90% der Asialofetuin Moleküle durch Lactose kompetitiert. Dieser Versuch verdeutlicht, daß mit dem Aufbau des ILA die Kohlenhydrat-Aktivität der Mistellektin-B Kette beschrieben wird.

### Beispiel 7: Vermessung der Volumenaktivität in Extraktpräparaten

Die Anwendbarkeit des ILA auf Extraktpräparate wurde hier beispielhaft mit dem Produkt Abnoba Viscum Mali D2, Ch.Bez.: 605 HIK-N-1 der Firma Abnoba, Pforzheim gezeigt. Das Präparat wurde einmal im Formulierungspuffer der Firma Abnoba und zum Vergleich dialysiert gegen den PVP-Puffer vermessen (Fig. 8). Eingesetzt wurden hier ebenfalls die Volumina 0/0.02/0.05/0.1/0.2/0.5/1/4/6/9/20/30 µl. Eine Auftragung der Absorption bei 490 nm gegen das steigende Volumen der beiden Proben zeigte, daß Extraktpräparate prinzipiell vermessen werden können. Ein signifkanter Einfluß der Puffer auf die Volumenaktivität konnte ausgeschlossen werden (Volumenaktivität in Formulierungspuffer: 1 Unit = 3.26 µl; entspr. 307 U/ml; Volumenaktivität in PVP-Puffer: 1 Unit = 3.15 µl; entspr. 317 U/ml).

### Literatur:

Barbieri, L., Battelli, G.M. und Stirpe, F. (1993): Ribosome-inactivating proteins from plants. Biochim. Biophys. Acta **1154**, 237-282
Beuth, J., Ko, H.L., Gabius, H.-J., Burrichter, H., Oette, K. and Pulverer, G. (1992): Behaviour of lymphocyte subsets and expression of activation markers in response to immunotherapy with galactoside-specific lectin from mistletoe in breast cancer patients. Clin. Investig. **70**, 658-661
Duk, M., Lisowska, E., Wu, H.J. und Wu, A.M. (1994): The biotin/avidin-mediated miorotiter plate leotin assay with the use of chemically modified glycoprotei ligand. Anal. Biochem. **221**, 266-272
Gablus, H.J., Walzel, H., Joshi, S.S., Kruip, J., Kojima, S., Gerke, V., Kratzim, H. und Gabius, S. (1992): The immunmodulatory β-galactoside-specific lectin from Mistletoe: Partial sequence analysis, cell and tissue binding and impact on intracellular biosignalling of monocytic leukemia cells. Anticancer Res. 1**2**, 669-676
Hajto, T., Hostanska, K. und Gabius, H.-J. (1989): Modular potency of the β-galactoside-specific lectin from Mistletoe extract (Iscador) on the host defense system in vivo in Rabbits and Patients. Cancer Res. **49**, 4803-4808
Harlow und Lane (1988): "Antibodies, A Laboratory Manual", CSH Press, Gold Spring Harbor, N.Y.
Hatakeyama, T., Murakami, K., Miyamoto, Y. und Yamasaki, N. (1996): An assay for lectin activity using miorotiter plate with chemically immobilized carbohydrates. Anal. Biochem. **237**, 188-192
Möckel, B., Schwarz, T., Zinke, H., Eck, J., Langer, M. und Lentzen, H. (1997): Effects of Mistletoe Lectin l on Human Blood Cell Lines and Peripheral Blood Cells. Drug Res. **47**, 1145-1151
Osborne et al. (1997): Curr. Opin. Chem. Biol. **1**, 5-9
Schöllhorn, V. (1993): Verfahren zur selektiven quantitativen Bestimmung der Konzentration von Lektinen, insbesondere Mistel-Lektinen. Deutsches Patent; DE 4123263 A1
Sharon, N. (1993): Lectin-carbohydrate complexes of plants and animals: an atomic view. TIBS **18**, 221-226.
Shimoda, T., und Funatsu, G. (1985): Binding of lactose and galactose to native and Iodinated Ricin D. J. Biol. Chem. **49**, 2125-2130
Stirpe, F., Barbieri, L., Battelli, M.G., Soria, M. und Lappi, D.A. (1992): Ribosome-inactivating proteins from plants: Present status and future prospects. Bio/Technology **10**, 405-412
Stull und Szoka (1995): Pharm. Res. **12**, 465-483
Tonevitzky, A.G., Rakamanova, V.A., Agapov, I.I., Shamshiev, A.T., Usacheva, E.A. und Prokoviev, S.A. (1995): The interactions of anti-ML-I monoclonal antibodies with isoforms of the lectin from viscum album. Immunol. Lett. **44**, 31-34
Vang, O., Pii Larsen, K. und Bog-Hansen, T.C. (1986): A new quantitative highly specific assay for lectinbinding activity. In: Lectins. Biology, Biochemistry, Clinical Biochemistry Vol. 5; Eds. Bog-Hansen, T.C. und van Driessche E. pp 637-644, W. de Gruyter, Berlin, NY
Yamashita, K., Umetsu, K., Suzuki, T. und Ohkura, T. (1992): Purification and characterization of a Neu5Ac α 2-6Gal β1-4 GlcNAc and HSO₃(-)- 6Gal β 1 -GlcNAc specific leotin in tuberous roots of *Trichosanthes japanica*. Biochemistry **31**, 11647-11650
Stahl, P.D. und Ezekowitz, R.A. (1998): The mannose receptor is a pattern recognition receptor involved in host defense. Curr. Opin. Immunol. 10, 50-55
Zanetta, J-P., Alonso, C. und Michalski J-C. (1996): Interleukin 2 is a lectin that associates its receptor with the T-cell receptor complex. Biochem. J. 318, 49-53

## Patentansprüche

1. Verfahren zur Identifizierung und/oder Quantifizierung einer ersten Substanz, welche Kohlenhydrat bindet, das die folgenden Schritte umfaßt:
(a) Zugabe einer zu testenden Probe zu einem Trägermaterial, wobei das Trägermaterial einen immobilisierten Rezeptor trägt, welcher einen Teil der ersten Substanz spezifisch bindet, der nicht in die Kohlenhydratbindung involviert ist;
(b) Zugabe einer zweiten Substanz, die einen Kohlenhydrat-Anteil enthält, wobei der Kohlenhydrat-Anteil durch die erste Substanz in einem Komplex gebunden wird und wobei der Komplex nachweisbar ist; und
(c) Nachweis des Komplexes.

2. Verfahren nach Anspruch 1, dadurch gekennzeichnet, daß die erste Substanz ein Lektin ist.

3. Verfahren nach Anspruch 2, dadurch gekennzeichnet, daß das Lektin ein pflanzliches Lektin ist.

4. Verfahren nach Anspruch 3, dadurch gekennzeichnet, daß das pflanzliche Lektin ein Mistellektin ist.

5. Verfahren nach einem der Ansprüche 1 bis 4, dadurch gekennzeichnet, daß die zweite Substanz, die einen Kohlenhydrat-Anteil enthält, ein Glykoprotein, ein Glykolipid, ein Kohlenhydrat oder ein synthetisches Substrat mit Kohlenhydrat-Anteil oder ein Neoglycoprotein, vorzugsweise Poly-N-Hydroxyethyl-N-SP-Biotinyl-N-SP-Glycosyl-Acrylamid ist.

6. Verfahren nach Anspruch 5, dadurch gekennzeichnet, daß das Glykoprotein Asialofetuin ist.

7. Verfahren nach Anspruch 5, dadurch gekennzeichnet, daß das synthetische Substrat mit Kohlenhydratanteil ein Neoglycoprotein, vorzugsweise Galactosyl-Rinderserumalbumin ist.

8. Verfahren nach einem der Ansprüche 1 bis 7, dadurch gekennzeichnet, daß der immobilisierte Rezeptor ein Antikörper, ein Aptamer oder ein Enzym ist.

9. Verfahren nach Anspruch 8, dadurch gekennzeichnet, daß der Antikörper ein monoklonaler Antikörper ist.

10. Verfahren nach einem der Ansprüche 1 bis 9, dadurch gekennzeichnet, daß die zweite Substanz, welche einen Kohlenhydrat-Anteil enthält, nachweisbar markiert ist.

11. Verfahren nach Anspruch 10, dadurch gekennzeichnet, daß die Markierung eine dritte Substanz ist, die eine vierte Substanz spezifisch bindet.

12. Verfahren nach einem der Ansprüche 1 bis 9, dadurch gekennzeichnet, daß der Nachweis vermittels eines zweiten Rezeptors erfolgt, welcher spezifisch an die zweite Substanz bindet.

13. Verfahren nach Anspruch 12, dadurch gekennzeichnet, daß der zweite Rezeptor nachweisbar markiert ist.

14. Verfahren nach Anspruch 12, dadurch gekennzeichnet, daß man für den Nachweis einen dritten Rezeptor zusetzt, wobei der zweite Rezeptor durch einen dritten Rezeptor gebunden wird und wobei der dritte Rezeptor, vorzugsweise durch ein daran gebundenes Enzym, nachweisbar markiert ist.

15. Verfahren nach Anspruch 11, dadurch gekennzeichnet, daß die dritte Substanz Biotin, Avidin oder Streptavidin ist.

16. Verfahren nach Anspruch 11, dadurch gekennzeichnet, daß die vierte Substanz Biotin, Avidin oder Streptavidin ist.

17. Verfahren nach Anspruch 12 oder 13, dadurch gekennzeichnet, daß der zweite und/oder dritte Rezeptor ein Antikörper, ein Aptamer oder ein Enzym ist.

18. Verfahren nach einem der Ansprüche 11 und 15 bis 17, dadurch gekennzeichnet, daß die vierte Substanz nachweisbar markiert ist.

19. Verfahren nach Anspruch 18, dadurch gekennzeichnet, daß die Markierung ein an die vierte Substanz gekoppeltes Enzym ist.

20. Verfahren nach einem der Ansprüche 10 und 13 bis 18, dadurch gekennzeichnet, daß die Markierung eine fluoreszierende, chemilumineszierende, biolumineszierende oder radioaktive Markierung oder colloidales Gold ist.

21. Verfahren nach einem der Ansprüche 1 bis 20, dadurch gekennzeichnet, daß der Nachweis durch Spektrometrie, Fluorimetrie, Luminometrie, Radiometrie, Potentiometrie oder enzymatisch / katalytisch erfolgt.

22. Verfahren nach einem der Ansprüche 14 und 17 bis 21, dadurch gekennzeichnet, daß das Enzym durch eine colorimetrische Substratumsetzung nachgewiesen wird.

23. Verfahren nach Anspruch 22, dadurch gekennzeichnet, daß das Enzym Meerrettich-Peroxidase oder alkalische Phosphatase ist.

24. Verfahren nach einem der Ansprüche 10 bis 23, dadurch gekennzeichnet, daß die zweite Substanz zusammen mit den weiteren Substanzen und/oder Rezeptoren in einem Schritt zugegeben wird.

25. Verfahren nach einem der Ansprüche 1 bis 24, dadurch gekennzeichnet, daß die erste Substanz in einem Rohextrakt nachgewiesen und/oder quantifiziert wird.

26. Verfahren nach einem der Ansprüche 1 bis 25, dadurch gekennzeichnet, daß eine Sättigung der Bindung der ersten Substanz unter einer Konzentration von 50 µg/ml, bevorzugt unter einer Konzentration von 5 µg/ml, stärker bevorzugt unter einer Konzentration von 2 µg/ml und besonders bevorzugt unter einer Konzentration von 0,9 µg/ml erreicht wird.

27. Verfahren nach einem der Ansprüche 1 bis 26, dadurch gekennzeichnet, daß das feste Trägermaterial eine Mikrotiterplatte, ein Teströhrchen oder ein Kügelchen (Bead) ist.

28. Test-Kit zur Ausführung des Verfahrens nach einem der Ansprüche 1 bis 27, enthaltend:
(a) einen Rezeptor, wie in einem der vorstehenden Ansprüche definiert, welcher einen Teil der ersten Substanz spezifisch bindet, der nicht in die Kohlenhydratbindung involviert ist;
(b) eine zweite Substanz, wie in einem der vorstehenden Ansprüche definiert; und
(c) eine Detektionssystem zum Nachweis der zweiten Substanz.

29. Test-Kit nach Anspruch 28, dadurch gekennzeichnet, daß
(a) der Rezeptor, weicher einen Teil der ersten Substanz spezifisch bindet, der nicht in die Kohlenhydratbindung involviert ist, ein monoklonaler Antikörper ist;
(b) die zweite Substanz biotinyliertes Asialofetuin ist, und
(c) das Detektionssystem mit Meerrettich-Peroxidase konjugiertes Avidin ist.

30. Test-Kit nach Anspruch 28 oder 29, dadurch gekennzeichnet, daß der Rezeptor an ein Trägermaterial immobilisiert ist.
